# EUROPÄISCHE PATENTANMELDUNG

(11) **EP 1 486 169 A2**
(43) Veröffentlichungstag der Anmeldung: **15.12.2004**
(21) Anmeldenummer: 04013707.7
(22) Anmeldetag: 11.06.2004
(51) Int. Cl.: A61B 7/00, A61B 9/00, A61B 8/06

(54) **Vorrichtung zur akustischen Untersuchung des Herzens eines Patienten**

(30) Priorität: 13.06.2003 DE 10327079; 06.12.2003 DE 20318977 U
(71) Anmelder: Brensing, Andreas, 21465 Reinbek (DE)
(72) Erfinder: Brensing, Andreas, Dr., 21465 Reinbek (DE); Rüter, Dirk, Dr., 22941 Bargteheide (DE); Eikhof,Arno, 21435 Stelle (DE); Henneberg, Jochen, 22952 Lütjensee (DE)
(74) Vertreter: Emmel, Thomas, Dipl.-Biol., Dr.

(57) **Zusammenfassung**

Die Erfindung betrifft eine Vorrichtung zur Untersuchung des Herzens eines Patienten (10), mit einer Aufnahmeeinrichtung (14), einer Speichereinrichtung und einer Auswerteeinrichtung (16), und ist dadurch gekennzeichnet, daß die Aufnahmeeinrichtung (14) zur Aufnahme von Herzsignalen ausgebildet ist, die durch ein definiertes akustisches Anregungssignal mit Signalfrequenzen in einem Bereich zwischen 5 kHz und 100 kHz ausgelöste Resonanzsignale des Herzens enthalten, und die Auswerteeinrichtung die aufgenommenen Herzsignale nach den Resonanzsignalen des Herzens ermittelnd, und die ermittelten Resonanzsignale nach darin enthaltenen Herzparametern auswertend ausgebildet ist.

## Beschreibung

Die Erfindung betrifft eine Vorrichtung der im Oberbegriff des Anspruches 1 genannten Art zur Untersuchung des Herzens eines Patienten.

Die physiologische Charakterisierung des Herzens eines Patienten ist in der medizinischen Diagnostik von großem Interesse. Interessierende Parameter sind dabei z.B. die Herzgröße, das Herzschlagvolumen, die Beeinträchtigung von Größe und Schlagvolumen durch weitere Faktoren wie z.B. die Atmung, die Ausmaße des den Herzbeutel umgebenden Fettgewebes oder der Zustand einer u.U. vorhandenen mechanischen Herzklappenprothese.

Aufgrund der guten schallleitenden Eigenschaften des Herzmuskelgewebes sind akustische Methoden bereits früh zur Herzuntersuchung verwendet worden.

Bei der Herzauskultation werden die durch Blutströmung und Herzklappenaktivität entstehenden Schallphänomene des Herzens von einem Arzt mit einem Stethoskop abgehört. Es handelt sich also um ein passives Verfahren. Die dabei berücksichtigten Frequenzen des Herzschalles liegen im Bereich zwischen 10 Hz und 2 kHz. Bei der prinzipiell ähnlichen Phonokardiographie werden die interessierenden Schallphänomene mit einem Mikrophon aufgenommen und zur leichteren Beurteilung in Form eines Phonokardiogrammes dargestellt.

Nachteil beider Verfahren ist, daß mit ihnen nur eine sehr beschränkte Anzahl von Parametern untersucht werden kann. Ein geübter Arzt kann damit anhand der jeweils typischen Geräusche z.B. Herzklappenfehler oder angeborene Herzfehler diagnostizieren. Informationen über die Herzgröße, das Herzschlagvolumen und andere Parameter liefern beide Methoden nicht. Außerdem ist für eine zutreffende Diagnose viel Erfahrung erforderlich und ein Patient kann die Verfahren nicht selber durchführen.

Bei dem Verfahren der Echokardiographie handelt es sich im Unterschied zu den zuvor beschriebenen Verfahren um eine aktive Methode, bei der Ultraschallwellen von außen auf den Körper in Herzrichtung eingestrahlt und die von den verschiedenen Gewebearten und ―schichten reflektierten Schallwellen aufgezeichnet werden. Die verwendeten Schallfrequenzen liegen bei der Echokardiographie im Bereich zwischen 1 und 20 MHz. Da Ultraschallwellen von verschiedenartigen Geweben unterschiedlich stark reflektiert werden, kann aus dem Muster der reflektierten Wellen ein Bild generiert werden, das Informationen über die Herzanatomie enthält. Aufgrund der verwendeten hohen Frequenzen liefert die Methode dabei eine relativ feine Bildauflösung. Die Echokardiographie wird daher überwiegend als bildgebende Methode zur Darstellung des Herzens eingesetzt. Zur zeitaufgelösten Charakterisierung physiologischer Parameter eignet sie sich nicht. Die Geräte sind außerdem von großer Bauart und ein Patient kann eine Echokardiographie nicht selber durchführen.

Bei der Herzperkussion handelt es sich ebenfalls um ein aktives Untersuchungsverfahren. Dabei klopft der Arzt die Körperoberfläche im Bereich des Thorax mit der Hand oder einem geeigneten Gerät ab, um aus den erzeugten Resonanzen, also den Eigenschwingungen der erschütterten Gewebe, auf die Ausdehnung und Beschaffenheit des Herzens zu schließen. Die dabei berücksichtigten Frequenzen des Herzschalles liegen wie bei den zuvor beschriebenen passiven Verfahren im Bereich zwischen 10 Hz und 2 kHz. Anders als bei dem ebenfalls aktiven Verfahren der Echokardiographie, bei dem die auf ein eingestrahltes Schallsignal von den Geweben reflektierten Schallwellen ausgewertet werden, wertet bei der Perkussion der Arzt die durch das Klopfen angeregten Resonanzen des Gewebes aus. Aufgrund der sehr schlechten zeitlichen Auflösung dieser manuellen Methode eignet sich das Verfahren lediglich für die Untersuchung der Beschaffenheit des interessierenden Gewebes. Eine zeitaufgelöste Charakerisierung physiologischer Parameter ist nicht möglich. Überdies läßt die Methode nur ungenaue Rückschlüsse zu, für eine zutreffende Diagnose ist sehr viel Erfahrung erforderlich und ein Patient kann das Verfahren nicht selbst durchführen.

Aufgabe der vorliegenden Erfindung ist es daher, eine Vorrichtung zur Untersuchung des Herzens zu schaffen, die die reproduzierbare Untersuchung weiterer physiologischer und ggf. morphologischer Parameter auf einfache Weise ermöglicht. Eine weitere Aufgabe ist es, die Vorrichtung so einfach zu gestalten, dass sie ein von einem Patienten selbst handhabbares Gerät zur Untersuchung des Patientenherzens ermöglicht.

Diese Aufgabe wird mit einer Vorrichtung gelöst, die die kennzeichnenden Merkmale des Anspruches 1 aufweist, sowie mit einem Gerät, das die Merkmale des Anspruches 30 aufweist.

Nach Anspruch 1 ist eine Vorrichtung zur Untersuchung des Herzens eines Patienten mit einer Aufnahmeeinrichtung, einer Speichereinrichtung und einer Auswerteeinrichtung vorgesehen, wobei die Aufnahmeeinrichtung zur Aufnahme von Herzsignalen ausgebildet ist, die durch ein definiertes akustisches Anregungssignal mit Signalfrequenzen in einem Bereich zwischen 5 kHz und 100 kHz ausgelöste Resonanzsignale des Herzens enthalten, und die Auswerteeinrichtung die aufgenommenen Herzsignale nach den Resonanzsignalen des Herzens ermittelnd, und die ermittelten Resonanzsignale nach darin enthaltenen Herzparametern auswertend ausgebildet ist.

Die Aufnahme und Bewertung solcher Resonanzsignale ermöglicht Rückschlüsse auf die Art und Umgebung des Herzens. Dabei macht man sich zunutze, daß die Resonanzfrequenz eines Gewebes u.a. abhängig ist von dessen Ausdehnung oder Größe. Eine relativ niedrige Resonanzfrequenz weist z.B. auf ein vergleichsweise großes Herz hin, während eine relativ hohe Resonanzfrequenz auf ein vergleichsweise kleines Herz hinweist.

Zudem ändern sich die jeweiligen Resonanzfrequenzen häufig mit der Zeit, z.B. durch die Volumenänderung des Herzbeutels während eines Atemzuges oder während der Systole. Diese Resonanzfrequenzänderungen können z.B. Informationen über die absolute Herzgröße, das Herzschlagvolumen oder die Änderung des Herzvolumens durch die Atmung liefern.

Der dabei interessierende Bereich der Resonanzfrequenzen entspricht dem Frequenzbereich des Anregungssignals. Als sehr aussagekräftig hat sich jedoch der Frequenzbereich zwischen 8 kHz und 40 kHz erwiesen. Besonders aussagekräftig ist der Frequenzbereich zwischen 10 kHz und 22 kHz. Es muß also nicht der gesamte Frequenzbereich von 5 kHz bis 100 kHz erfaßt werden, ein Teilbereich, z.B. einer der oben genannten Teilbereiche, enthält die benötigten Resonanzfrequenzen des Herzens. Die technischen Mittel zum Messen im fraglichen kHz-Bereich sind im Stand der Technik bekannt, und bedürfen daher keiner näheren Erläuterung.

Akustische Resonanzverfahren sind in der Medizin z.B. aus der Lungendiagnostik bekannt (Blechschmidt RA, Werthschützky R (2002): Akustische Diagnostik der Lunge. Technisches Messen 69(4), 194). Hier wird über einen elektrodynamischen Aktor, der einen Stößel aufweist und auf den Brustkorb eines Patienten aufgelegt wird, ein breitbandiges Schallsignal in den Thorax eingespielt und mit einem stethoskopähnlichen Mikrofon, das auf der entgegengesetzten Seite des Thorax auf den Brustkorb aufgelegt wird, der transmittierte Schall aufgezeichnet. Aus der Dämpfung bzw. Resonanzverstärkung bestimmter Frequenzen kann so eine Aussage über Veränderungen in Lungenvolumen und Lungendichte, die z.B. krankhafter Natur sein können, getroffen werden. Der dabei zur Anwendung kommende Frequenzbereich liegt im unteren Bereich des hörbaren Spektrums zwischen 100 und 500 Hz, wobei der Bereich um 320 Hz besonders aussagekräftig ist. Der bei dem Verfahren verwendete elektrodynamische Aktor kann zwar Schwingungen großer Amplitude auf den Brustkorb aufbringen, ist aber nur für die Erzeugung solch niedriger Frequenzen geeignet. Mit den verwendeten hörbaren Frequenzen können jedoch lediglich Resonanzsignale luftgefüllter Organe aufgenommen werden, da nur diese sehr niedrige Resonanzfrequenzen haben. Aus diesem Grund findet das beschriebene Verfahren ausschließlich Anwendung bei der Charakterisierung der Organe des Atemtraktes.

Die akustischen Eigenschaften (Dichte, Elastizitätsmodul, akustische Impedanz, Dämpfung, Schallgeschwindigkeit) von Luft auf der einen Seite und Wasser bzw. Gewebe auf der anderen Seite unterscheiden sich stark. Daher ergeben sich für die Erzeugung, Aufnahme und Auswertung von Resonanzsignalen bei nicht luftgefüllten Organen andere technische und theoretische Grundvoraussetzungen.

Insbesondere liegen bei nicht luftgefüllten Organen die Resonanzfrequenzen um ein Vielfaches höher.

Eine Übertragung des beschriebenen Lungendiagnostikverfahrens auf nicht luftgefüllte Organe ist daher nicht möglich. Erst die Merkmale des erfindungsgemäßen Anspruchs 1 erlauben die Auswertung von Resonanzphänomenen bei einem Herzen. Dabei werden im Gegensatz zu dem beschriebenen Verfahren Frequenzen aus dem nahen Ultraschallbereich verwendet, die damit um mindestens eine Größenordnung höher liegen als bei dem beschriebenen Lungendiagnostikverfahren. Der besonders aussagekräftige Bereich liegt sogar um einen Faktor 50 höher. Diese Frequenzen lassen sich mit den Methoden aus dem beschriebenen Verfahren nicht erzeugen bzw. auswerten, und sind bisher im Stand der Technik nicht herzdiagnostisch genutzt worden.

Wie bereits geschildert, nimmt die erfindungsgemäße Vorrichtung durch ein definiertes akustisches Anregungssignal ausgelöste Resonanzsignale im Bereich des Herzens auf, ermittelt daraus die Resonanzsignale des Herzens und bewertet diese. In einer bevorzugten Ausgestaltung der Erfindung ist vorgesehen, daß die Vorrichtung eine Schallerzeugungseinrichtung zur Ausstrahlung des Anregungssignals aufweist. Diese Schallerzeugungseinrichtung kann bevorzugt ein piezokeramisches Element sein. Solche Elemente weisen in dem genannten Anregungsfrequenzbereich einen höheren Wirkungsgrad auf als z.B. Spulenlautsprecher, die sich besser für niedrigere Frequenzen eignen. Nichtsdestotrotz sind auch Spulenlautsprecher oder andere Ultraschallquellen als Schallerzeugungseinrichtungen im Rahmen der vorliegenden Erfindung geeignet.

In vorteilhafter Ausgestaltung ist vorgesehen, als Anregungssignal Rauschsignale, schnell durchstimmbare monofrequente Signale (Sweepsignale) und/oder breitbandige Impulssignale zu verwenden. Allen diesen Signaltypen ist gemein, daß sie innerhalb eines gegebenen Zeitraumes ein breites Frequenzband abdekken. Wie weiter unten dargelegt, eignen sich alle drei Signaltypen zur Erzeugung der gewünschten Resonanzen des Herzens. Konstante monofrequente bzw. schmalbandige Signale eignen sich dagegen nur bedingt, da die Wahrscheinlichkeit besteht, daß die Resonanzfrequenzen der jeweiligen Gewebe nicht im monofrequenten bzw. schmalbandigen Anregungssignal enthalten sind und die betreffenden Gewebe daher nicht oder nur sehr schwach in Resonanz gebracht werden können.

Als Anregungssignal kann alternativ oder zusätzlich aber auch ein von einer anderen Schallquelle erzeugtes Signal verwendet werden. Dieses Signal kann z.B. in einer vorteilhaften Ausgestaltung das von einem künstlichen Herzklappenimplantat im Körper des Patienten verursachte Geräusch sein, z.B. das Öffnungsgeräusch, das Scharniersignal und/oder das Schließgeräusch des Herzklappenimplantates. Bei allen diesen Geräuschen handelt es sich um sehr kurze Impulssignale mit einer Dauer von weniger als 5 ms, die eine hohe spektrale Bandbreite aufweisen und daher Resonanzen im Bereich des Herzens zu erzeugen vermögen. Untersuchungen des Anmelders haben erstmals gezeigt, daß die bei Trägern von künstlichen Herzklappenimplantaten wahrzunehmenden typischen Schließgeräusche, die eine Dauer von bis zu 20 ms haben können, nur zu einem kleinen Teil aus dem eigentlichen Schließgeräusch der Klappe und zum weitaus größeren Teil aus dem Resonanzgeräusch des umgebenden Herzgewebes bestehen. Das eigentliche Geräusch eines Herzklappenimplantates kann also in gleicher Weise wie ein von einer Schallerzeugungseinrichtung außerhalb des Körpers erzeugtes Schallsignal zur Anregung von Resonanzen im Herzraum verwendet werden. Dessen ungeachtet können aber auch andere körpereigene oder körperfremde Schallquellen verwendet werden, solange sie geeignet sind, Resonanzen der interessierenden Organe hervorzurufen.

In einer besonders vorteilhaften Ausgestaltung ist dabei vorgesehen, aus den Eigenschaften des durch das Geräusch des künstlichen Herzklappenimplantats angeregten Resonanzsignals zusätzlich Aussagen zu der Art und dem Zustand des Implantats zu ermitteln. Diesem Vorgehen liegt zugrunde, daß sich im Laufe der Zeit die Klangeigenschaften einer künstlichen Herzklappe durch Abnutzung oder Thrombosierung ändern können, so daß sich die Intensität und/oder die spektrale Zusammensetzung des kurzen Anregungssignals ändert. Infolgedessen ändern sich auch die Eigenschaften der durch die Anregungssignale erzeugten akustischen Resonanzsignale des Herzraumes. Ein stark thrombosiertes Implantat erzeugt z.B. im Vergleich zu einem unbeeinträchtigten Implantat ein Resonanzsignal mit vermindertem hochfrequenten Anteil (> 2 kHz) und mit lediglich eingipfeligem Amplitudenspektrum.

Durch Vergleich eines durch ein unbeeinträchtigtes Implantat erzeugten Referenz-Resonanzsignals mit dem Resonanzsignal, das durch ein Implantat angeregt wurde, dessen Zustand fraglich ist, kann also der Zustand des zu untersuchenden Implantats beurteilt und so z.B. ein Thrombus diagnostiziert werden. Ein solcher Thrombus kann sich lösen und einen Gefäßverschluß verursachen, so daß durch rechtzeitige Diagnose eine geeignete Therapie eingeleitet werden kann.

In weiteren vorteilhaften Ausgestaltungen, die unabhängig von der Art der resonanzanregenden Schallquelle verwirklicht sein können, ist vorgesehen, daß die Vorrichtung das Ausklingverhalten, das Amplitudenspektrum, das Spektrogramm und/oder die Phasenlage der Resonanzsignale auswertet. Dabei enthält das Ausklingverhalten des Resonanzsignals u.a. Informationen über die akustische Isolation des Herzraumes. Eine stark ausgeprägte akustische Isolation kann z.B. ein Hinweis auf einen hohen Anteil an Fettgewebe im Herzraum sein. Das Amplitudenspektrum des Resonanzsignals enthält z.B. Informationen über effektive Volumina von Organen im Herzraum. Ein Resonanzsignal hoher Frequenz läßt z.B. auf ein relativ kleines Herz schließen, während ein Resonanzsignal niedriger Frequenz auf ein relativ großes Herz schließen läßt. Das Spektrogramm des Resonanzsignals enthält z.B. Informationen über zeitabhängige Volumenänderungen von Organen im Herzraum, und läßt daher z.B. Aussagen über das Herzschlagvolumen oder - indirekt über die atmungsbedingte Änderung des Herzvolumens - über die effektive Atmungstiefe zu.

Als besonders vorteilhaft zur Auswertung haben sich dabei solche Signalanteile des Resonanzsignals herausgestellt, die sich im Rhythmus der Herzbewegung und/oder der Atmung in der Intensität, Phase oder ihrer Frequenz verändern.

In vorteilhaften Ausgestaltungen ist vorgesehen, daß die Aufnahmeeinrichtung und gegebenenfalls die Schallerzeugungseinrichtung in reproduzierbarer Weise an verschiedenen Punkten der Körperoberfläche aufgebracht werden kann. Durch die reproduzierbare Aufbringung an verschiedenen Punkten der Körperoberfläche können verschiedene Schwingungsrichtungen und Schwingungsmoden, die u.U. jeweils andere, sich gegebenenfalls ergänzende Informationen liefern können, angeregt bzw. ausgewertet werden. Bevorzugt werden die genannten Einrichtungen dabei an eindeutig identifizierbaren Punkten der Körperoberfläche positioniert. Es kann aber auch vorgesehen sein, daß die genannten Einrichtungen mit Hilfe von Hilfseinrichtungen wie z.B. Gurten oder Korsetts unabhängig von besonders auffälligen Punkten reproduzierbar auf der Körperoberfläche positioniert werden.

Nach einem weiteren Aspekt der Erfindung ist die Vorrichtung auch zur Aufnahme und Bewertung der Herzleistung eines Patienten ausgebildet.

Die Herzleistung, die z.B. als Herzzeitvolumen (HZV) ausgedrückt werden kann, ist eine zentrale Größe zur Diagnose von Patienten, insbesondere Sportlern oder Patienten mit Herzinsuffizienz. Die Herzleistung kann durch leicht erfassbare Größen wie Puls, Blutdruck oder EKG nur unzureichend charakterisiert werden, da diese keine Auskunft über die Strömungsgeschwindigkeit des Blutes im Aortenbogen geben.

Aus diesem Grunde werden für die Bestimmung der Herzleistung aufwendige Methoden verwendet, wie z.B. die Indikatorverdünnungsmethode, bei der die Verdünnung eines intravenös oder durch Inhalation zugeführten Indikators, radioaktiv markierten Stoffes oder Gases gemessen wird, oder die Radionuklid-Angiokardiographie. Beide Methoden stellen für den Patienten eine hohe Belastung dar, insbesondere bei Patienten mit Herzinsuffizienz, und eignen sich daher nicht für Routineuntersuchungen.

In der ärztlichen Praxis werden inzwischen Herzleistungs-Messgeräte verwendet, die eine Ultraschall-Messeinrichtung aufweisen. Eine solche Vorrichtung ist zum Beispiel aus der DE 36 250 41 bekannt.

Diese Geräte arbeiten nach dem Doppler-Prinzip, d.h. sie strahlen über eine Sendeeinrichtung Ultraschalltöne in den Körper des Patienten ein und nehmen zeitversetzt die von den Geweben reflektierten Ultraschalltöne auf. Treffen die ausgesandten Ultraschalltöne auf sich bewegende Objekte, wie z.B. Blutzellen, so werden sie mit einer leicht versetzten Frequenz reflektiert. Objekte, die sich in Richtung der Sendeeinrichtung bewegen, reflektieren einen Ultraschallton mit leicht erhöhter Frequenz, während Objekte, die sich von der Sendeeinrichtung wegbewegen, Töne mit leicht erniedrigter Frequenz reflektieren. Dieses Phänomen ist als Doppler-Effekt bekannt. Das Messgerät nimmt die reflektierten Töne auf und kann aus der Differenz zwischen ausgesandter und wiederaufgenommener Frequenz die Geschwindigkeit der betreffenden Objekte berechnen, so auch die Fließgeschwindigkeit des Blutes.

Die genannten Geräte sind durchweg so aufgebaut, dass sie eine stationäre Auswerteeinheit aufweisen sowie eine Bedieneinheit, die die Ultraschall-Sendeeinrichtung enthält und die direkt auf den Körper des Patienten aufgesetzt wird. Die Auswerteeinheit weist u.a. eine Recheneinrichtung, eine Anzeigeeinrichtung und eine Ausgabeeinrichtung auf. Die gesamte Vorrichtung eignet sich aufgrund ihrer Größe lediglich für den stationären Einsatz, z.B. in einer Arztpraxis oder in einer Klinik, nicht jedoch zur autonomen Verwendung durch den Patienten selbst, z.B. zu Hause oder am Arbeitsplatz. Dies ist gerade bei Patienten mit Herzinsuffizienz sehr nachteilig.

Ein wesentliches Ziel der vorliegenden Erfindung ist es daher auch, eine Vorrichtung zur Aufnahme und Bewertung der Herzleistung eines Patienten zu schaffen, die sich für den mobilen Einsatz eignet und mit der ein Patient selbständig und regelmäßig eine Bewertung seiner Herzleistung vornehmen kann, um z.B. im Falle einer auftretenden Herzinsuffizienz einen Arzt hinzuzuziehen.

Erfindungsgemäß ist die Vorrichtung zur Aufnahme und Bewertung der Herzleistung eines Patienten mit einer Ultraschall-Sendeeinrichtung, einer Empfängereinrichtung und einer Auswerteeinrichtung ausgebildet. Dabei ist vorgesehen, dass die Ultraschall-Sendeeinrichtung ein von Körperbereichen des Patienten reflektierbares Signal emittiert, und die Empfängereinrichtung das reflektierte Signal aufnimmt. Die Auswerteeinrichtung ermittelt dann als Ausgangsmeßwert eine auf dem Doppler-Effekt beruhende Frequenzverschiebung zwischen emittiertem und reflektiertem Signal und berechnet aus dem Ausgangsmeßwert die Herzleistung des Patienten.

Dem Anmelder gelang es erstmals, alle für die Messung sowie die Auswertung erforderlichen Komponenten in einem tragbaren, netzunabhängigen und vom Patienten bedienbaren Handgerät zu integrieren.

Dies ist nur durch die bislang nicht vorgenommene Kombination von seit neuestem verfügbaren Prozessor- und Speicherbausteinen, die sich durch hohe Kapazität und geringe Ausmaße sowie geringen Stromverbrauch auszeichnen, und intelligenten Algorithmen, mit deren Hilfe die anfallenden Datenmengen reduziert sowie die erforderliche Rechenleistung reduziert werden können, möglich. Die Kapazität der verwendeten Prozessor- und Speicherbausteinen alleine würde bei Verwendung konventioneller Algorithmen für die zu erledigenden Rechen- bzw. Speicheraufgaben nicht ausreichen.

Der von der Auswerteeinrichtung berechnete Ausgangsmeßwert ist noch kein direktes Maß für die Herzleistung. In einer bevorzugten Ausgestaltung der Erfindung ist daher vorgesehen, dass die Auswerteeinrichtung unter Berücksichtigung mindestens eines patientenspezifischen Proportionalitätsfaktors aus dem ermittelten Ausgangsmeßwert die Herzleistung des Patienten direkt berechnet. Der patientenspezifische Faktor kann z.B. durch die eingangs erwähnte Indikatorverdünnungs- oder Radionuklidmethode bestimmt werden.

In einer weiteren bevorzugten Ausgestaltung der Erfindung ist vorgesehen, dass die Auswerteeinrichtung aus dem ermittelten Ausgangsmeßwert zunächst eine zeitabhängige, die Herzleistung des Patienten beschreibende Größe berechnet, und dann mit Hilfe des patientenspezifischen Proportionalitätsfaktors die Herzleistung des Patienten berechnet.

Eine besonders bevorzugte zeitabhängige Größe ist die Fließgeschwindigkeit des Blutes im Aortenbogen des Patienten. Dabei ermittelt die Auswerteeinrichtung die Fließgeschwindigkeit, indem sie die durch den Dopplereffekt verursachte Frequenzverschiebung zwischen den eingestrahlten Signalen und den von den im Aortenbogen flottierenden Partikeln reflektierten Signalen auswertet. Bei diesen Partikeln handelt es sich überwiegend um rote Blutkörperchen. Die Differenz zwischen eingestrahlten und reflektierten Signalen ist ein Maß für die Geschwindigkeit der Partikel und damit für die Fließgeschwindigkeit des Blutes. In diesem Falle dient als Proportionalitätsfaktor die. Querschnittsfläche des Aortenbogens, die z.B. durch Echokardiographie oder durch eine Katheteruntersuchung ermittelt werden kann. Das Produkt aus Fließgeschwindigkeit [cm s⁻¹] und Querschnitssfläche [cm²] ergibt das Herzzeitvolumen [cm³ s⁻¹], das, wie oben dargestellt, ein Maß für die Herzleistung ist.

Es sind jedoch auch andere zeitabhängige Größen zur Beschreibung der Herzleistung denkbar, die dann natürlich entsprechend andere Proportionalitätsfaktoren verlangen.

In jedem der genannten Fälle kann vorgesehen sein, daß die Auswerteeinrichtung z.B. beim Arzt einmalig durch Eingabe des oder der Proportionalitätsfaktoren für den jeweiligen Patienten kalibriert werden muß.

Bevorzugt ist vorgesehen, dass die von der Sendeeinrichtung emittierten Signale im Frequenzbereich zwischen 2 und 10 MHz liegen. Dieser Bereich bietet einen optimalen Kompromiß zwischen der Ultraschall-Eindringtiefe und der räumlichen Auflösung.

Als Ultraschall-Sendeeinrichtung und als Empfängereinrichtung wird bevorzugt ein Piezoelement verwendet. Dabei kann entweder ein Element verwendet werden, das abwechselnd - z.B. über einen Pulsgeber gesteuert - als Sendeeinrichtung und als Empfängereinrichtung fungiert, oder es werden zwei Elemente verwendet, die jeweils nur eine der beiden Funktionen wahrnehmen. Im ersten Fall sendet das Piezoelement ein Signal aus, verstummt dann und nimmt während eines bestimmten, darauf folgenden Zeitintervalls die reflektierten Signale auf. Im zweiten Fall werden die beiden Piezo-Elemente wechselseitig aktiv.

Durch die Dauer des Aufnahmeintervalls und seine relative zeitliche Lage zum Sendeintervall kann der Tiefenbereich der Messung, d.h. also - von der Empfangseinrichtung aus gesehen - die minimale, die mittlere und die maximale Entfernung der Körperbereiche, deren Reflektionssignale ausgewertet werden sollen, definiert werden.

In einer weiteren bevorzugten Ausgestaltung der erfindungsgemäßen Vorrichtung weist die Auswerteeinrichtung einen A/D-Wandler und einen digitalen Signalprozessor auf.

Dabei ist besonders bevorzugt vorgesehen, dass die Vorrichtung über die Auswerteeinrichtung das aufgenommene, von Körperbereichen des Patienten reflektierte Signal digitalisiert, einer Fourier-Transformation unterzieht, und aus dem erhaltenen Spektrogramm mittels Hüllkurvenbildung eine zeitabhängige Größe ermittelt, die die Herzleistung des Patienten beschreibt. Dabei legt der Signalprozessor eine Hüllkurve über die Frequenzen des Spektrogramms. Die mittlere Höhe der Hüllkurve ergibt eine der Herzleistung des Patienten proportionale Größe.

Die erfindungsgemäße Vorrichtung weist in einer bevorzugten Ausgestaltung eine Speichereinrichtung zum Speichern der ermittelten zeitabhängigen Größe auf. In einer weiteren bevorzugten Ausgestaltung ist dabei vorgesehen, dass die Vorrichtung eine zweite Speichereinrichtung zum Speichern des oder der Proportionalitätsfaktoren aufweist.

In einer bevorzugten Ausgestaltung der Erfindung ist vorgesehen, dass die Ultraschall-Sendeeinrichtung und die Empfängereinrichtung in einem vorstehenden Bereich des Gehäuses der Vorrichtung angeordnet sind, wobei der vorstehende Bereich des Gehäuses so gestaltet ist, dass er leicht und positionssicher in reproduzierbarer Weise an einem Bereich des Patienten direkt oberhalb des Thorax am Hals des Patienten ansetzbar ist. Durch dieses Merkmal wird eine einfache, genaue und reproduzierbare Positionierung der Ultraschall-Sendeeinrichtung und der Empfängereinrichtung sichergestellt. Damit wird eine gute Signalqualität sowie die Reproduzierbarkeit der erhaltenen Meßergebnisse gewährleistet.

Besonders bevorzugt ist dabei vorgesehen, dass das Gehäuse der Vorrichtung und sein vorstehender Bereich so gestaltet sind, dass die Ultraschall-Sendeeinrichtung bei an den Hals des Patienten angelegtem Zustand in Richtung des Aortenbogens des Patienten strahlt. Dabei können das Gehäuse der Vorrichtung und sein vorstehender Bereich so gestaltet sein, daß sich eine Positionierung an anderen Bereichen des Körpers aufgrund der Formgebung schon intuitiv ausschließt.

Dieses Merkmal gewährleistet, dass ein großer Anteil der von der Sendeeinrichtung ausgesandten Signale von im Aortenbogen flottierenden Partikeln reflektiert und von der Empfängereinrichtung wieder aufgenommen wird. Überdies wird gewährleistet, dass die Ausbreitungsrichtung der Schallwellen nahezu tangential zum Scheitelpunkt des Aortenbogens und/oder parallel zu der durch den Aortenbogen definierten Ebene angeordnet ist. Die Ausbreitungsrichtung der Schallwellen und die Bewegungrichtung der im Bereich des Scheitelpunkts des Aortenbogens im Blut flottierenden Partikel sind daher zueinander nahezu parallel. Die durch den Dopplereffekt bewirkte Frequenzverschiebung ist bei diesen Richtungsverhältnissen maximal. Das von der Empfängereinrichtung aufgenommene Signal stellt somit ein genaues Maß für die Fließgeschwindigkeit des Blutes im Aortenbogen des Patienten dar und weist eine hohe Amplitude sowie ein günstiges Signal-Rausch-Verhältnis auf.

In einer vorteilhaften Ausgestaltung ist vorgesehen, daß die Vorrichtung batteriebetrieben ist, und alle erforderlichen Einrichtungen der erfindungsgemäßen Vorrichtung in einem Handgerät angeordnet sind. Ein solches Handgerät kann z.B. in der ambulanten Diagnose oder vom Patienten zuhause verwendet werden. Es ist dem Patienten dadurch möglich, Untersuchungen zu seiner Herzleistung außerhalb der Klinik oder der ärztlichen Praxis und ohne Anwesenheit eines Arztes durchzuführen. Die Untersuchungsintervalle können so erheblich verdichtet werden. Der Patient gewinnt damit an Sicherheit, gleichzeitig sinkt die Belastung durch Untersuchungen außer Haus. Insbesondere Patienten mit Herzinsuffizienzen gewinnen so erheblich an Lebensqualität.

In einer weiteren vorteilhaften Ausgestaltung ist vorgesehen, daß die Vorrichtung eine Ausgabeeinrichtung aufweist, die ein Auswertungsergebnis wiedergibt. Eine solche Ausgabeeinreichtung kann z.B. ein LCD-Display oder eine akustische Sprachausgabe sein. Es können ein, mehrere oder alle Ergebnisse dargestellt werden, es kann auch zwischen verschiedenen Ergebnisanzeigen hin- und hergeschaltet werden.

In einer weiteren vorteilhaften Ausgestaltung ist vorgesehen, daß die Vorrichtung eine Schnittstelle aufweist, über die sie mit einem Rechner, einem Telekommunikationsgerät oder einem Drucker kommunizieren kann. Über diese Schnittstelle lassen sich z.B. Meßdaten von der Vorrichtung auf einen PC übertragen, über das Telekommunikationsgerät an eine Auswertungszentrale weiterleiten oder direkt mit einem geeigneten Drucker ausdrucken. Weiterhin können über die Schnittstelle Betriebssoftware oder Betriebsparameter auf die Vorrichtung überspielt werden. Dieses Merkmal erleichtert z.B. den Datenaustausch zwischen der Vorrichtung und einem PC-gestützten Diagnose- oder Archivierungssystem. Überdies ist die Möglichkeit der Ferndiagnose durch eine qualifizierte Person eröffnet. Insbesondere kann so im Falle einer Krisensituation schnell Hilfe geleistet oder herangeführt werden. Eine solche Schnittsstelle kann z.B. ein GSM-, IrDA-, USB-, RS 232-, FireWire- oder Bluetooth-Interface oder eine andere Schnittstelle nach dem derzeitigen oder kommenden Stand der Technik sein.

In einer weiteren vorteilhaften Ausgestaltung ist vorgesehen, daß die Vorrichtung eine Einrichtung zur Ableitung eines EKGs aufweist. Hierdurch erhöht sich der Nutzwert für den Patienten, und er kann in einer Krisensituation sowohl seine Herzleistung als auch sein EKG messen bzw. messen lassen, ohne dass weitere Zeit verloren geht.

In einer weiteren bevorzugten Ausgestaltung ist vorgesehen, dass die Vorrichtung eine akustische Signalgebeeinrichtung und/oder eine Timereinrichtung aufweist. Mittels dieser Merkmale kann die Vorrichtung den Patienten z.B. an die regelmäßige Benutzung der Vorrichtung erinnern oder den Beginn und das Ende des Meßintervalls anzeigen, oder dem Patienten die korrekte Positionierung der Vorrichtung signalisieren.

In weiteren bevorzugten Ausgestaltungen der erfindungsgemäßen Vorrichtung ist vorgesehen, dass die Vorrichtung eine Einrichtung zur Aufbewahrung von Medikamenten, ein mobiles Telekommunikationsgerät, eine Einrichtung zur Funkalarmierung eines Rettungs- oder Hilfsdienstes oder eine Satellitennavigationseinrichtung aufweist.

Bei der Messung von Fließgeschwindigkeit nach dem Doppler-Prinzip ist zu beachten, dass das Geschwindigkeitsprofil einer laminar in einem Rohr strömenden Flüssigkeit einen paraboliden Verlauf annimmt. Randnahe Flüssigkeitsschichten fließen folglich mit geringerer Geschwindigkeit als zentrale Flüssigkeitsschichten.

In einer besonderen Ausgestaltung der erfindungsgemäßen Vorrichtung kann vorgesehen sein, dass die Vorrichtung über die bereits beschriebene Einstellung des Empfangsintervalls so justiert werden kann, dass sie entweder nur Signale auswertet, die von einer schmalen Flüssigkeitsschicht reflektiert werden, oder dass sie alle die Signale auswertet, die von Flüssigkeitsschichten aus dem gesamten Querschnitt des Aortenbogens reflektiert werden.

In beiden Fällen muß bei der Berechnung der Herzleistung bzw. einer zeitabhängigen Größe wie der mittleren Fließgeschwindigkeit des Blutes im Aortenbogen das Geschwindigkeitsprofil durch Verwendung eines weiteren, geeigneten Proportionalitätsfaktors berücksichtigt werden. Dieser Faktor kann durch theoretische Berechnungen oder experimentell, z.B. durch Echokardiografie oder eine Kathetheruntersuchung, bestimmt und im Gerät gespeichert werden. Er ist überdies abhängig vom Abstand und der Dicke des Bereichs, dessen Reflektionssignale die Vorrichtung auswertet.

In den Abbildungen ist die Erfindung beispielsweise und schematisch dargestellt. Darin zeigen:
- Fig. 1:: eine Vorrichtung zur Aufnahme und Auswertung der Resonanzen des Herzens eines Patienten in einer ersten Ausführungsform;
- Fig. 2:: eine Vorrichtung zur Aufnahme und Auswertung der Resonanzen des Herzens eines Patienten in einer zweiten Ausführungsform;
- Fig. 3:: eine mit einer Vorrichtung gemäß Fig. 2 experimentell ermittelte Resonanz des Herzens eines Patienten;
- Fig. 4:: das Amplitudenspektrum des in Fig. 3 dargestellten Klangsignals bei gefüllter Lunge des Patienten;
- Fig. 5:: das Amplitudenspektrum eines zu Fig. 3 ähnlichen Klangsignals bei entleerter Lunge des Patienten;
- Figs. 6, 7 und 8:: in schematischer Form Frequenz-Zeit-Diagramme verschiedener Anregungssignale;
- Figs. 9, 10 und 11:: schematische Frequenz-Zeit-Diagramme möglicher Resonanzsignale des Herzens nach Anregung mit dem in Fig. 6 gezeigten Rauschsignal;
- Fig. 12:: eine Resonanzaufnahme des aktiven menschlichen Herzens eines Patienten als Sonagramm bei Anregung mit einem Signal gemäß Fig. 6;
- Fig. 13:: eine hypothetische Resonanzaufnahme des Herzens in einem FrequenzZeit-Diagramm bei Anregung mit einem Signal gemäß Fig. 7;
- Fig. 14:: eine Resonanzaufnahme des aktiven menschlichen Herzens eines Patienten als Sonagramm bei Anregung mit einem Signal gemäß Fig. 8;
- Fig. 15:: den schematischen Aufbau einer erfindungsgemäßen Vorrichtung gemäß einer weiteren Ausführungsvariante;
- Fig. 16:: die Positionierung der Vorrichtung der Fig. 15 am Körper eines Patienten; und
- Fig. 17:: ein schematische Darstellung des Auswertungsablaufes in der Vorrichtung der Fig. 15.

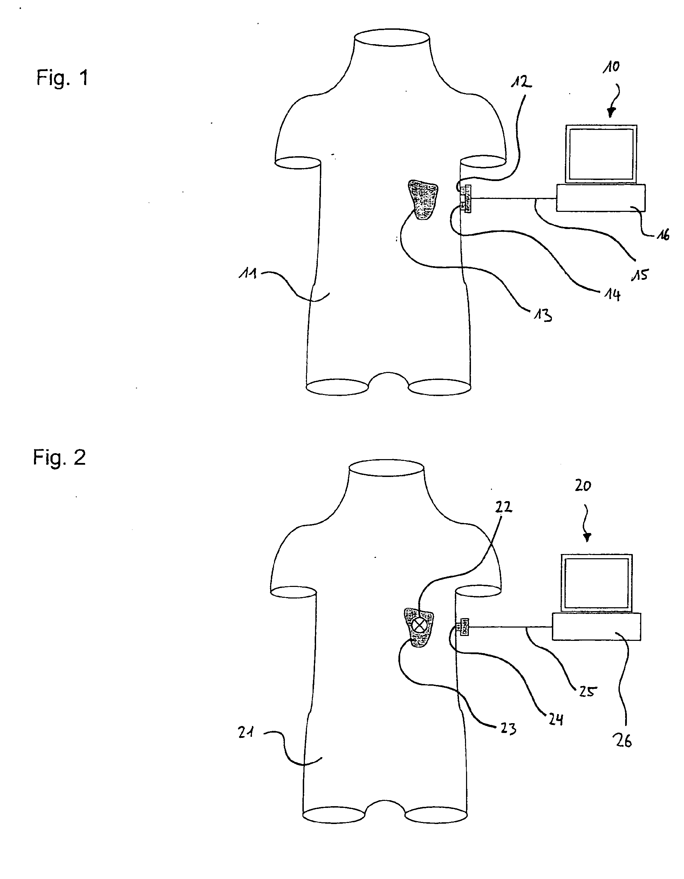

Fig. 1 zeigt eine Vorrichtung 10 zur Aufnahme und Auswertung der Resonanzen des Herzens eines Patienten 11, mit einer auf den Brustkorb des Patienten 11 aufgesetzten Schallerzeugungseinrichtung 12, die im dargestellten Fall aus einem piezokeramischen Element besteht, Schallwellen in den Herzraum sendet und dabei charakteristische Resonanzfrequenzen des Herzens 13 anregt. Die Resonanzschallwellen werden von einer ebenfalls auf den Brustkorb aufgesetzten Aufnahmeeinrichtung 14 wieder aufgenommen und über eine Kabelverbindung 15 einer Auswerteeinrichtung 16 zugeführt, die in der Abbildung in Form eines PC dargestellt ist. Die Auswerteeinrichtung bearbeitet und analysiert genau diejenigen Frequenzen, die in Korrelation mit dem Herzschlag und gegebenenfalls auch mit der Atmung in der Intensität und/oder in der Frequenz- bzw. Phasenlage variieren. Diese veränderlichen Signale sind mit Gewissheit mit dem Herz verkoppelt und enthalten Informationen über das Herz und seine Umgebung.

Fig. 2 zeigt eine Vorrichtung 20 zur Aufnahme und Auswertung der Resonanzen des Herzens eines Patienten 21, die jedoch im Unterschied zu der in Fig. 1 gezeigten Vorrichtung 10 keine Schallerzeugungseinrichtung 12 aufweist. In diesem Fall trägt der Patient ein künstliches Herzklappenimplantat 22, dessen breitbandiges und impulsartiges Geräusch Resonanzen des Herzens 23 erzeugt. Diese werden mit einer auf den Brustkorb aufgesetzten Aufnahmeeinrichtung 24 aufgenommen und über eine Drahtverbindung 25 der Auswerteeinrichtung 26 zugeführt.

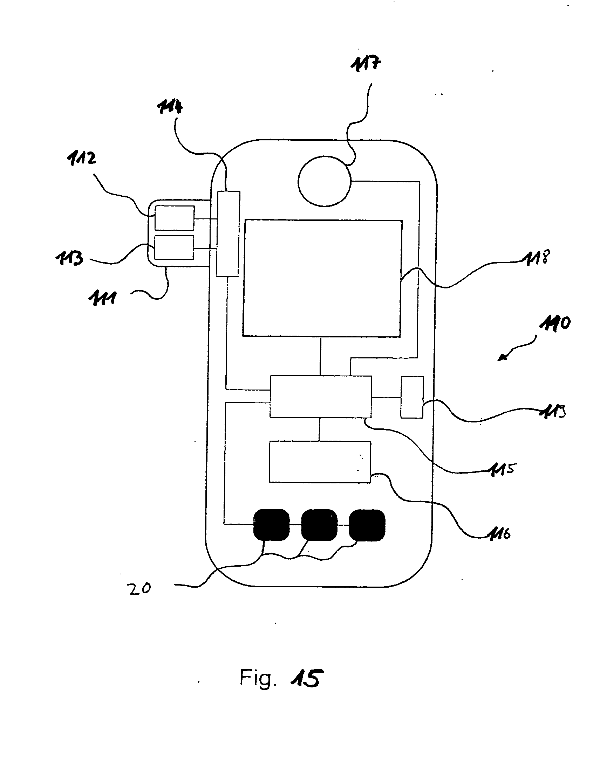

Die Auswerteeinrichtung 16, 26 kann anders als in Fig. 1 und 2 dargestellt in Form eines kompakten Handgerätes ausgebildet sein, in dem Aufnahmeeinrichtung und gegebenenfalls auch Schallerzeugungseinrichtung eingebaut sind. Das Gerät kann z.B. dem in Fig. 15 dargestellten Beispiel entsprechen. In Abweichung von Fig. 1 und 2 kann statt der Drahtverbindung 15, 25 eine Funkverbindung verwendet werden.

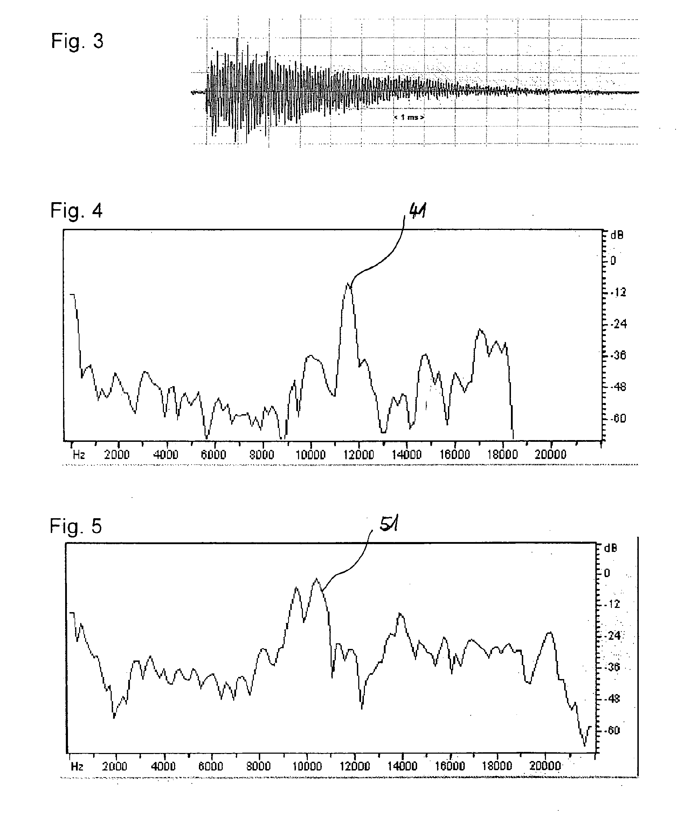

Fig. 3 zeigt den Zeitverlauf einer mit einer Vorrichtung gemäß Fig. 2 experimentell gemessenen Resonanz hoher Güte des Herzens eines Patienten. Die Resonanz wurde durch das Schließgeräusch eines harten Herzklappenimplantates angeregt. Die hohe Intensität und die geringe Dämpfung des Signals mit einem deutlich erkennbaren Eigenfrequenzanteil mit etwa 11 kHz lassen auf ein intaktes Implantat und eine gute akustische Isolation des Herzens schließen. Deutlich hervorgehoben sei an dieser Stelle, daß das gemessene Signal nicht vom Implantat herrührt, vielmehr ist das Implantat nur der Auslöser dieser Resonanzen. Direkte Signalanteile vom Implantat sind hier praktisch kaum vorhanden. Die Lage und Höhe der Eigenfrequenzen gestatten gleichwohl detaillierte Rückschlüsse auf die Art und den Zustand des Herzklappenimplantates.

Fig. 4 zeigt das Amplitudenspektrum eines solchen in Fig. 3 dargestellten Herzsignals bei gefüllter Lunge. Das Spektrum weist ein Resonanzmaximum 41 bei etwa 11,5 kHz auf. Die Resonanzfrequenz eines Hohlkörpers wie dem Herzen ist umgekehrt proportional zum Volumen des Organs. Ein großvolumiges Herz hat also eine niedrigere Resonanzfrequenz als ein kleinvolumiges. Dies wird anschaulich aus Fig. 5.

Fig. 5 stellt das Spektrum eines ähnlichen Herzsignals bei entleerter Lunge dar. Das Spektrum weist ein Resonanzmaximum 51 bei etwa 10,5 kHz auf. Es ist also gut erkennbar, daß sich die Resonanzfrequenzen mit der atmungsbedingten Kompression bzw. Dekompression des Herzens verändern.

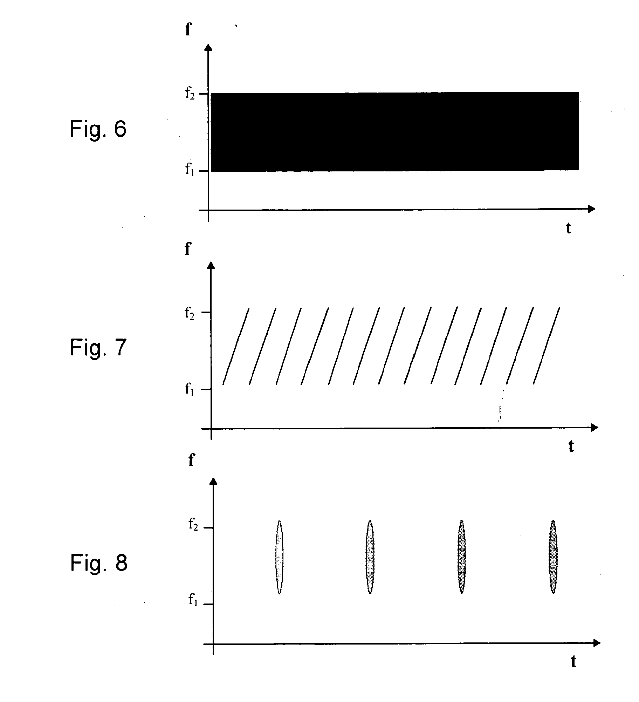

In den Figuren. 6, 7 und 8 sind in Form von Frequenz-Zeit-Diagrammen schematisch verschiedene in Frage kommende, durch die Schallerzeugungseinrichtung 12 ausstrahlbare Anregungssignale dargestellt. Aufgrund der charakteristischen Resonanzeigenschaften der interessierenden Gewebe ist der verwendete Frequenzbereich durch die Frequenzen f₁ und f₂ eingegrenzt.

Dabei zeigt Fig. 6 ein Rauschsignal, Fig. 7 ein periodisch wiederholtes Sweepsignal und Fig. 8 ein breitbandiges, periodisch wiederholtes Impuls- oder Klicksignal. Letzteres kann z.B. im Gegensatz zu den zuvor gezeigten Anregungssignalen auch von einem künstlichen Herzklappenimplantat im Körper eines Patienten herrühren.

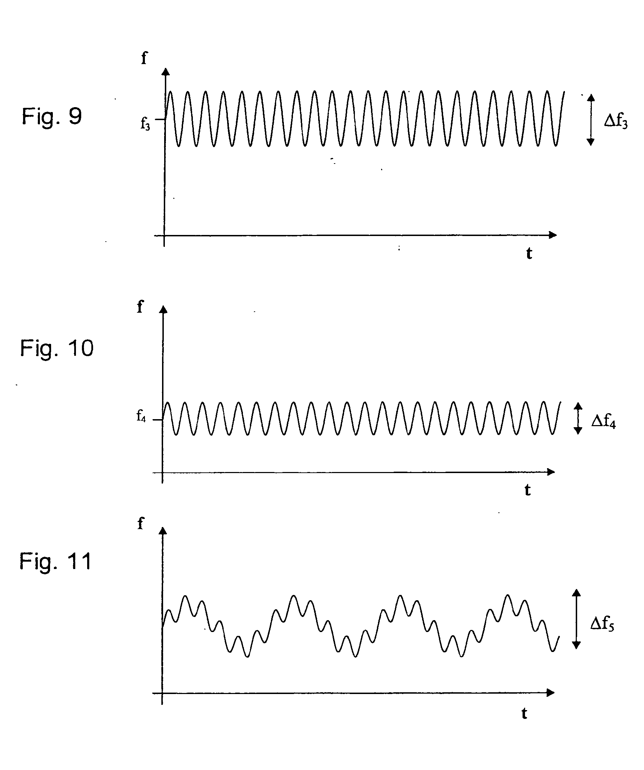

In den Figuren. 9, 10 und 11 sind in Form von Frequenz-Zeit-Diagrammen schematisch verschiedene mögliche Resonanzsignale des Herzens nach Anregung mit dem in Fig. 6 gezeigten Rauschsignal dargestellt.

Fig. 9 zeigt ein Signal mit sich periodisch ändernder Resonanzfrequenz. Die mittlere Resonanzfrequenz f₃ und die periodisch auftretende Frequenzänderung mit der Amplitude Δf₃ liegen relativ hoch. Der Kehrwert der Resonanzfrequenz f₃ ist ein Maß für die Größe des resonanten Volumens, also z.B. des effektiven Herzvolumens, während der Quotient Δf₃/f₃ ein Maß für die relative Größenänderung des resonanten Volumens ist, z. B. aufgrund der zyklischen Herzkontraktion.

Fig. 10 stellt ein Signal mit einer relativ niedrigen mittleren Resonanzfrequenz f₄ und einer ebenfalls relativ niedrigen periodischen Frequenzänderung mit der Amplitude Δf₄ dar. Es kann daher im Vergleich zu Fig. 9 auf ein größeres effektives Herzvolumen und ein geringeres relatives Herzschlagvolumen geschlossen werden.

In Fig. 11 ist schematisch ein Signal des Herzraumes mit periodischer, herzschlagbedingter Resonanzfrequenzänderung dargestellt, dem eine langsamere Frequenzänderung der Amplitude Δf₅ überlagert ist. Die überlagerte Frequenzänderung geht auf eine besonders ausgeprägte Atemaktivität eines Patienten zurück, die im Atemrhythmus eine langsame Volumenänderung des Herzens verursacht, die der eigentlichen, herzschlagbedingten Volumenänderung des Herzens überlagert ist. Die Figs. 4 und 5 stellen gleichsam Momentaufnahmen dieses Phänomens dar.

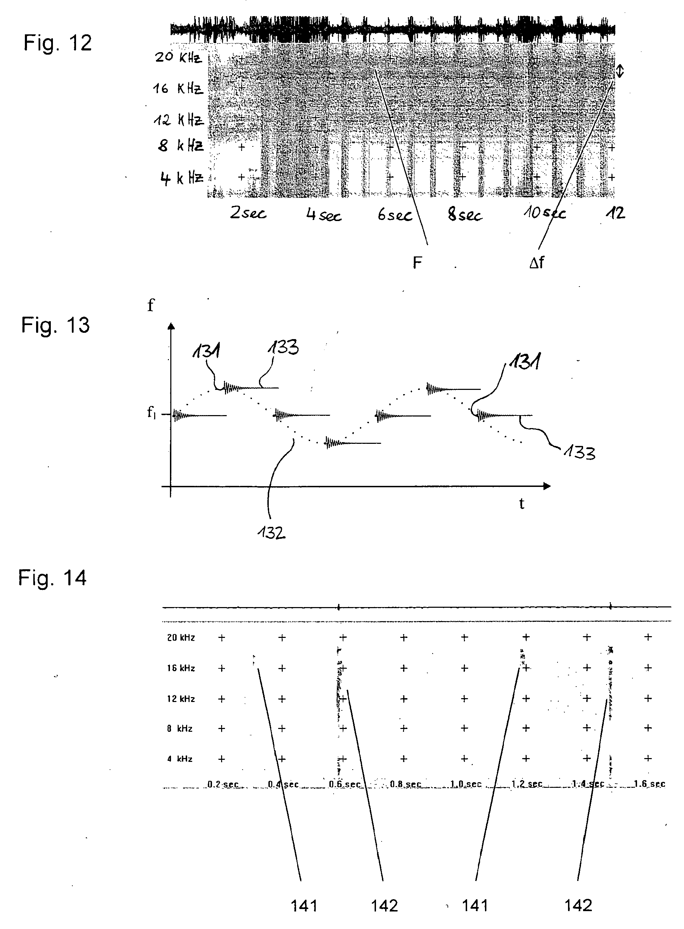

In Fig. 12 ist eine reale Resonanzaufnahme eines aktiven menschlichen Herzens eines Patienten in Form eines Sonagrammes dargestellt. Dabei handelt es sich um Frequenz-Zeit-Diagramme, die überdies noch eine Amplitudeninformation enthalten, deren Darstellung über den Grauwert erfolgt. Dabei codiert ein starker Grauwert eine große Amplitude und ein schwacher Grauwert eine schwache Amplitude.

Die externe Anregung des in Fig. 12 gezeigten Resonanzsignals erfolgte mit einem wie in Fig. 6 dargestellten Rauschsignal. Dabei weist das Resonanzsignal eine periodisch ihre Frequenz ändernde Komponente mit einer mittleren Frequenz f von etwa 18 kHz und einer Amplitude Δf von etwa 1 kHz auf. Durch Eichung mit auf andere Weise gewonnen Daten lassen sich aus den Werten für f und Δf Meßgrößen für das effektive Herzvolumen und das relative Herzschlagvolumen gewinnen.

In Fig. 13 ist eine hypothetische Resonanzaufnahme des Herzens in einem Frequenz-Zeit-Diagram dargestellt. Anders als zuvor erfolgte die Anregung hier nicht mit einem wie in Fig. 6 dargestellten Rauschsignal, sondern mit einem wie in Fig. 7 dargestellten Sweepsignal. Dabei wird in den interessierenden Geweben nicht kontinuierlich eine Resonanz erzeugt, sondern immer nur dann, wenn das anregende Sweepsignal gerade die jeweils passende Resonanzfrequenz durchläuft. Auf diese Weise erhält man diskrete Resonanzantworten 131, aus denen sich ein kontinuierliches Signal 132 rekonstruieren läßt, das dem in Fig. 12 gezeigten Resonanzsignal bei F entspricht. Dabei ist zu beachten, daß die Wiederholfrequenz des Sweepsignals mindestens doppelt so hoch sein sollte wie die Frequenz der Volumenänderung des interessierenden Organs, um eine ausreichende Meßauflösung zu erreichen. Im Falle des Herzens, das typischerweise mit einer Frequenz zwischen 40 und 200 min⁻¹ sein Resonanzvolumen ändert, sollte die Wiederholfrequenz des Sweepsignals also mindestens 400 min⁻¹ bzw. 6.7 Hz betragen.

Im Vergleich zur Rauschanregung (Figur 12) bringt die Sweepanregung (Figur 13) jedoch zwei Vorteile mit sich. Da die Schallerzeugungseinrichtung zu einem definierten Zeitpunkt immer nur eine genau definierte Frequenz abstrahlt und nicht ein breitbandiges Rauschsignal, kann das Anregungssignal wesentlich intensitätsstärker abgestrahlt werden. Man erhält daher auch ein sehr viel stärkeres Resonanzsignal. Aufgrund der Diskontinuität des Anregungssignals enthalten die Resonanzantworten 131 überdies eine abklingende Komponente 133. Das Maß der Dämpfung dieser abklingenden Komponente wird dabei durch die akustische Isolation des sich in Resonanz befindenden Organs bestimmt. Im Falle des Herzens kann z.B. angenommen werden, daß ein hoher Anteil an Fettgewebe im Herzraum das Herz gegenüber dem Brustraum stark akustisch isoliert, so daß die Resonanzantworten 131 wenig gedämpft werden und daher lange nachklingen. Ein geringer Anteil an Fettgewebe kann im Gegensatz dazu zu einer starken Dämpfung der Resonanzantworten 131 führen. Die durch Sweepsignale angeregten Resonanzantworten enthalten also u.U. eine zusätzliche, unter diagnostischen Gesichtspunkten interessante Information zum Anteil des Fettgewebes im Brustraum.

In Fig. 14 ist wiederum eine reale Resonanzaufnahme eines aktiven menschlichen Herzens eines Patienten in Form eines Sonagrammes dargestellt. Die externe Anregung erfolgte dabei mit einem wie in Fig. 8 dargestellten breitbandigen Klicksignal, das von einem künstlichen Herzklappenimplantat im Körper des Patienten herrührt. Es sind daher wie bei Anregung mit Sweepsignalen nur diskontinuierliche Resonanzantworten 141, 142 zu gewinnen.

Die in Fig. 12 und Fig. 14 gezeigten Aufnahmen erfolgten an zwei verschiedenen, aber ähnlich gebauten Patienten, wobei im Fall der Figur 14 der Patient ein künstliches Herzklappenimplantat trug. Auffallend ist beim Vergleich beider Abbildungen zunächst die Ähnlichkeit der Frequenzlage der Resonanzen aus Fig. 12 sowie der Resonanzantworten 141 aus Fig. 14, die jeweils im Bereich zwischen 16 und 20 kHz liegen. Es spricht daher viel dafür, daß die Resonanzantworten 141 aus Figur 14 auf denselben Gewebetypus zurückgehen wie die Resonanzen aus Figur 12.

Die in Fig. 14 gezeigten Resonanzantworten 141 wurden durch das Öffnungsräusch des Klappenimplantats angeregt, während die Resonanzantworten 142 durch das Schließgeräusch des Implantats angeregt wurden. Es ist gut erkennbar, daß die Schließsignale 142 lauter, aber auch undifferenzierter sind als die Öffnungssignale 141.

Aus den Eigenschaften des durch das Geräusch des künstlichen Herzklappenimplantats angeregten Resonanzsignals lassen sich wie weiter oben beschrieben Aussagen zu der Art und dem Zustand des Implantats machen. Ein stark thrombosiertes Implantat erzeugt z.B. im Vergleich zu einem unbeeinträchtigten Implantat ein Resonanzsignal mit vermindertem hochfrequenten Anteil (> 2 kHz) und mit lediglich eingipfeligem Amplitudenspektrum.

Aufgrund der besseren Differenzierung der Öffnungssignale 141 eignen sich die hierdurch angeregten Resonanzen besser zur Bewertung des Herzklappenzustandes als die Resonanzen, die durch die Schließsignale 142 angeregt wurden. Ähnliches gilt für das hier nicht dargestellte Scharniersignal eines künstlichen Herzklappenimplantats. Dabei handelt es sich um ein relativ schwaches, aber qualitativ hochwertiges Signal zur Anregung von Herzresonanzen, das entsprechend gut geeignet ist zur Charakterisierung der Scharnierqualität.

Fig. 15 zeigt ein Handgerät 110 mit einem Ausleger 111, der einen Sender 112 und einen Empfänger 113 für Ultraschallwellen umschließt. Sender 112 und Empfänger 113 stehen unter der Kontrolle einer Steuereinrichtung 114, die nicht dargestellt einen Ultraschalloszillator, einen Sende- und einen Empfangsverstärker sowie einen Mischer aufweist. Der Ausleger 111 ist geometrisch so ausgestaltet, dass er leicht und positionssicher im Bereich direkt oberhalb des Thorax am Hals des Patienten angesetzt werden kann. Die aus dem Aortenbogen reflektierte Schalleistung des Senders ist wegen der Bewegung des Blutes um die Dopplerfrequenz versetzt. Das Signal des Empfängers wird in der Steuereinrichtung 114 verstärkt und mit der Sendefrequenz aus dem Oszillator im Mischer heruntergemischt.

Das Signal wird in einem Signalprozessor 115 digitalisiert und einer Fourier-Transformation unterzogen, die ein zeitabhängiges Amplitudenspektrum (Sonagramm) erstellt. Der Signalprozessor legt dann eine Hüllkurve über die Frequenzen maximaler Amplitude des Sonagramms. Die mittlere Höhe der Hüllkurve ist dem Herzzeitvolumen proportional. Der oder die in einer Speichereinrichtung 116 abgelegte Proportionalitätsfaktoren sind patientenspezifisch und ergeben sich z.B. aus einer einmaligen Referenzmessung mit Echokardiografie. Bei den Proportionalitätsfaktoren kann es sich z.B. um den Durchmesser der Aorta und/oder die Geschwindigkeitsprofile des individuellen Patienten handeln.

Der Patient erhält bei der Anwendung des Handgerätes akustische Hilfssignale aus einem Schallgeber 117 zur Unterstützung der sicheren und korrekten Positionierung des Auslegers 111. Im Messbetrieb wird aus dem Schallgeber 117 z.B. das typische Dopplersignal ausgegeben.

Auf einem Display 118 werden als Maß für die Herzleistung die Geschwindigkeitsverläufe, die Maximalgeschwindigkeiten sowie das aktuelle Herzzeitvolumen dargestellt. Die Ergebnisse können mit Datierung in der Speicherinrichtung 116 abgelegt und jederzeit wieder aufgerufen werden.

Zudem verfügt das Gerät über eine Datenschnittstelle 119 zur Transferierung der patientenspezifischen Herzleistungs-Ergebnisse. Diese Schnittstelle kann z.B. ein GSM-, IrDA-, USB-, RS 232-, FireWire- oder Bluetooth-Interface oder eine andere Schnittstelle nach dem derzeitigen oder kommenden Stand der Technik sein. Die Bedienung des Handgerätes erfolgt über die Tasten 120.

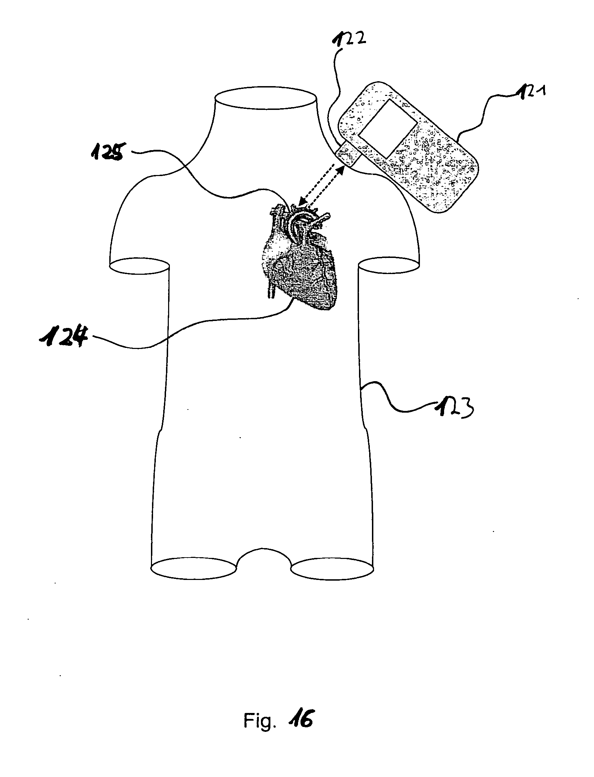

Fig. 16 zeigt die Positierung eines Handgeräts 121 mit einem Ausleger 122 im Bereich des Halsansatzes eines Patienten 123 mit einem Herz 124 und einem Aortenbogen 125. Durch die spezifische Bauart wird sichergestellt, dass - wie durch die Pfeile angedeutet - die Ausbreitungsrichtung der aus dem Ausleger 122 emittierten Schallwellen nahezu tangential zum Scheitelpunkt des Aortenbogens 125 und/oder parallel zu der durch den Aortenbogen 125 definierten Ebene angeordnet ist.

Die Ausbreitungsrichtung der Schallwellen und die Bewegungrichtung der im Bereich des Scheitelpunkts des Aortenbogens im Blut flottierenden Partikel sind daher zueinander parallel. Die durch den Dopplereffekt bewirkte Frequenzverschiebung ist bei diesen Richtungsverhältnissen maximal. Das erhaltene Meßsignal weist daher ein optimales Signal-Rausch-Verhältnis auf.

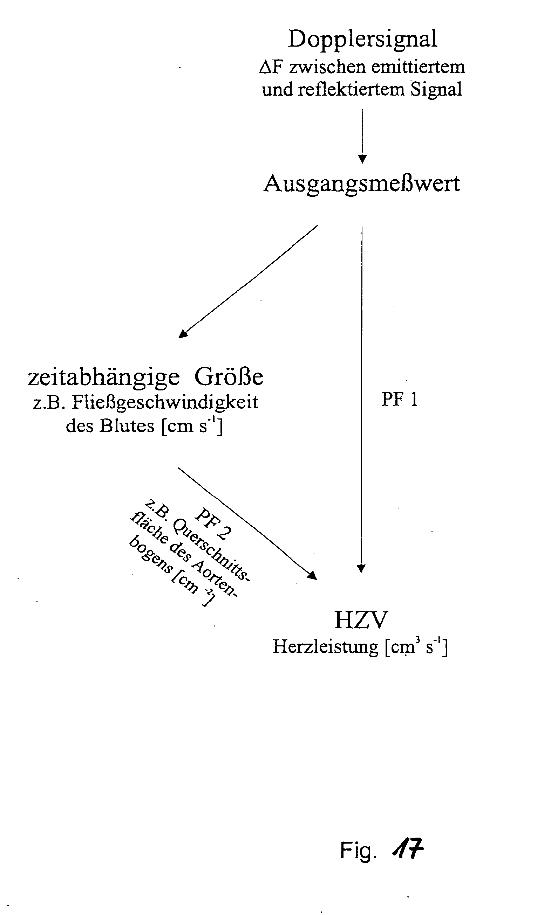

Fig. 17 zeigt schematisch den Auswertungsablauf in der oben beschriebenen Vorrichtung.

In den Figuren nicht dargestellt ist ein Gerät, das die beiden bisher anhand der Figuren 1 bis 17 getrennt beschriebenen Geräte zur Untersuchung des Herzens einerseits und zur Messung der Herzleistung andererseits und deren Eigenschaften und die damit möglichen Meß- und Auswerteverfahren vereint in ein Gesamtgerät. Ein solches Gesamtgerät könnte z.B. wie in Fig. 15 aussehen, z.B. mit einem zweiten Messkopf 111,122 , oder mit in dem einen Kopf 111 der Figur 15 angeordneten weiteren Aufnahme- und Schallerzeugungseinrichtung. Einige der Einrichtungen könnten auch von beiden Meß- und Auswerteverfahen genutzt werden, z.B. Speicher, Auswerteinrichtung oder Anzeigeinrichtung. Die beiden bisher getrennt beschriebenen Messverfahren könnten z.B. durch entsprechende Wahl des Bedieners am Gerät eingestellt und dazwischen umgeschaltet werden. Es könnten aber auch beide Messverfahren gleichzeitig durchgeführt werden. Die getrennte Beschreibung der beiden Messverfahren diente lediglich der Übersichtlichkeit.

## Patentansprüche

1. Vorrichtung zur Untersuchung des Herzens eines Patienten, mit einer Aufnahmeeinrichtung, einer Speichereinrichtung und einer Auswerteeinrichtung, **dadurch gekennzeichnet, daß** die Aufnahmeeinrichtung zur Aufnahme von Herzsignalen ausgebildet ist, die durch ein definiertes akustisches Anregungssignal mit Signalfrequenzen in einem Bereich zwischen 5 kHz und 100 kHz ausgelöste Resonanzsignale des Herzens enthalten, und die Auswerteeinrichtung die aufgenommenen Herzsignale nach den Resonanzsignalen des Herzens ermittelnd, und die ermittelten Resonanzsignale nach darin enthaltenen Herzparametern auswertend ausgebildet ist.

2. Vorrichtung nach Anspruch 1, **dadurch gekennzeichnet, daß** die Vorrichtung eine Schallerzeugungseinrichtung zur Ausstrahlung des Anregungssignals aufweist.

3. Vorrichtung nach einem der vorhergehenden Ansprüche, **dadurch gekennzeichnet, daß** ein piezokeramisches Element die Schallerzeugungseinrichtung ausbildet.

4. Vorrichtung nach einem der vorhergehenden Ansprüche, **dadurch gekennzeichnet, daß** das Anregungssignal als Rauschsignal, schnell durchstimmbares monofrequentes Signal (Sweepsignale) und/oder Impulssignal ausgebildet ist.

5. Vorrichtung nach Anspruch 1, **dadurch gekennzeichnet, daß** die Vorrichtung ein von einem künstlichen Herzklappenimplantat im Körper des Patienten erzeugtes Geräusch als Anregungssignal verwendend ausgebildet ist, wobei dieses Geräusch ein Öffnungsgeräusch, ein Scharniersignal und/oder ein Schließgeräusch des Herzklappenimplantates sein kann.

6. Vorrichtung nach Anspruch 5, **dadurch gekennzeichnet, daß** die Vorrichtung die Resonanzsignale des Herzraumes nach Art und Zustand des künstlichen Herzklappenimplantates auswertend ausgebildet ist.

7. Vorrichtung nach einem der vorhergehenden Ansprüche, **dadurch gekennzeichnet, daß** die Vorrichtung das Ausklingverhalten, das Amplitudenspektrum und/oder das Spektrogramm der Resonanzsignale auswertend auswertend ausgebildet ist.

8. Vorrichtung nach einem der vorhergehenden Ansprüche, **dadurch gekennzeichnet, daß** die Vorrichtung insbesondere solche Frequenzen des Resonanzsignals zur Auswertung heranzieht, die sich im Rhythmus der Herzbewegung und/oder Atmung in der Intensität, Phase oder Frequenz verändern.

9. Vorrichtung nach einem der vorhergehenden Ansprüche, **dadurch gekennzeichnet, daß** die Vorrichtung so gestaltet ist, daß die Aufnahmeeinrichtung in reproduzierbarer Weise an verschiedenen Punkten der Körperoberfläche aufbringbar ist.

10. Vorrichtung nach einem der Ansprüche 1 bis 4 und 7 bis 9, **dadurch gekennzeichnet, daß** die Vorrichtung so gestaltet ist, daß die Schallerzeugungseinrichtung in reproduzierbarer Weise an verschiedenen Punkten der Körperoberfläche aufbringbar ist.

11. Vorrichtung nach einem der vorhergehenden Ansprüche, **dadurch gekennzeichnet, dass** sie zur Aufnahme und Bewertung der Herzleistung eines Patienten ausgebildet ist, eine Ultraschall-Sendeeinrichtung zum Aussenden eines von Körperbereichen des Patienten reflektierbaren Signals sowie eine Empfängereinrichtung zur Aufnahme eines reflektierten Signals aufweist, und die Auswerteeinrichtung eine auf dem Doppler-Effekt beruhende Frequenzverschiebung zwischen emittiertem und reflektiertem Signal als Ausgangsmeßwert ermittelnd und die Herzleistung des Patienten ausgehend von dem Ausgangsmesswert berechnend ausgebildet ist.

12. Vorrichtung nach Anspruch 11, **dadurch gekennzeichnet, dass** die Auswerteeinrichtung unter Berücksichtigung mindestens eines patientenspezifischen Proportionalitätsfaktors aus dem ermittelten Ausgangsmeßwert die Herzleistung des Patienten direkt berechnet.

13. Vorrichtung nach Anspruch 11, **dadurch gekennzeichnet, dass** die Auswerteeinrichtung aus dem ermittelten Ausgangsmeßwert zunächst eine zeitabhängige, die Herzleistung des Patienten beschreibende Größe berechnet, und dann mit Hilfe mindestens eines patientenspezifischen Proportionalitätsfaktors die Herzleistung des Patienten berechnet.

14. Vorrichtung nach Anspruch 13, **dadurch gekennzeichnet, dass** die ermittelte zeitabhängige Größe die Fließgeschwindigkeit des Blutes im Aortenbogen des Patienten und der Proportionalitätsfaktor die Querschnittsfläche des Aortenbogens ist.

15. Vorrichtung nach einem der Ansprüche 11 bis 14, **dadurch gekennzeichnet, dass** die Sendeeinrichtung Ultraschallwellen im Frequenzbereich zwischen 2 und 10 MHz emittiert.

16. Vorrichtung nach einem der Ansprüche 11 bis 15, **dadurch gekennzeichnet, dass** die Ultraschall-Sendeeinrichtung und/oder die Empfängereinrichtung ein Piezoelement aufweisen.

17. Vorrichtung nach einem der Ansprüche 11 bis 16, **dadurch gekennzeichnet, dass** die Auswerteeinrichtung einen A/D-Wandler und einen digitalen Signalprozessor aufweist.

18. Vorrichtung nach einem der Ansprüche 11 bis 17, **dadurch gekennzeichnet, dass** die Vorrichtung in der Auswerteeinrichtung das aufgenommene, von Körperbereichen des Patienten reflektierte Signal digitalisiert, einer Fourier-Transformation unterzieht und aus dem erhaltenen Spektrogramm mittels Hüllkurvenbildung die zeitabhängige Größe ermittelt, die die Herzleistung des Patienten beschreibt.

19. Vorrichtung nach einem der Ansprüche 11 bis 18, **dadurch gekennzeichnet, dass** die Vorrichtung eine Speichereinrichtung zum Speichern der ermittelten zeitabhängigen Größe aufweist.

20. Vorrichtung nach einem der Ansprüche 11 bis 19, **dadurch gekennzeichnet, dass** die Vorrichtung eine zweite Speichereinrichtung zum Speichern des oder der Proportionalitätsfaktoren aufweist.

21. Vorrichtung nach einem der Ansprüche 11 bis 20, **dadurch gekennzeichnet, dass** die Ultraschall-Sendeeinrichtung und die Empfängereinrichtung in einem vorstehenden Bereich des Gehäuses der Vorrichtung angeordnet sind, wobei der vorstehende Bereich des Gehäuses der Vorrichtung so gestaltet ist, dass er leicht und positionssicher in reproduzierbarer Weise an einem Bereich des Patienten direkt oberhalb des Thorax am Ansatz des Halses ansetzbar ist.

22. Vorrichtung nach einem der Ansprüche 11 bis 21, **dadurch gekennzeichnet, dass** das Gehäuse der Vorrichtung und sein vorstehender Bereich so gestaltet sind, dass die Ultraschall-Sendeeinrichtung bei an den Hals des Patienten angelegtem Zustand in Richtung des Aortenbogens des Patienten strahlt.

23. Vorrichtung nach einem der vorhergehenden Ansprüche, **dadurch gekennzeichnet, daß** die Vorrichtung eine Ausgabeeinrichtung aufweist, die ein Auswertungsergebnis darstellt.

24. Vorrichtung nach einem der vorhergehenden Ansprüche, **dadurch gekennzeichnet, dass** die Vorrichtung in einem tragbaren, stromnetzunabhängigen und vom Patienten bedienbaren Handgerät untergebracht ist.

25. Vorrichtung nach einem der vorhergehenden Ansprüche, **dadurch gekennzeichnet, daß** die Vorrichtung eine Schnittstelle aufweist, über die sie mit einem Rechner, einem Telekommunikationsgerät oder einem Drucker kommunizieren kann.

26. Vorrichtung nach einem der vorhergehenden Ansprüche, **dadurch gekennzeichnet, dass** in die Vorrichtung zusätzlich ein mobiles Telekommunikationsgerät integriert ist.

27. Vorrichtung nach einem der vorhergehenden Ansprüche, **dadurch gekennzeichnet, daß** die Vorrichtung eine Einrichtung zur Ableitung eines EKGs aufweist.

28. Vorrichtung nach einem der vorhergehenden Ansprüche, **dadurch gekennzeichnet, dass** die Vorrichtung eine akustische Signalgebeeinrichtung und/oder eine Timereinrichtung aufweist.

29. Vorrichtung nach einem der vorhergehenden Ansprüche, **dadurch gekennzeichnet, dass** die Vorrichtung eine Einrichtung zur Aufbewahrung von Medikamenten aufweist.

30. Vorrichtung nach einem der vorhergehenden Ansprüche, **dadurch gekennzeichnet, dass** die Vorrichtung zusätzlich ein mobiles Telekommunikationsgerät, eine Einrichtung zur Funkalarmierung eines Rettungs- oder Hilfsdienstes aufweist oder eine Satellitennavigationseinrichtung aufweist.

31. Tragbares, stromnetzunabhängiges und vom Patienten bedienbares Handgerät, in der eine Vorrichtung zur Aufnahme und Bewertung der Herzleistung eines Patienten untergebracht ist, mit einer Ultraschall-Sendeeinrichtung zum Aussenden eines von Körperbereichen des Patienten reflektierbaren Signals, einer Empfängereinrichtung zur Aufnahme eines reflektierten Signals und einer Auswerteeinrichtung zur Ermittlung einer auf dem Doppler-Effekt beruhenden Frequenzverschiebung zwischen emittiertem und reflektiertem Signal als Ausgangsmeßwert und zur Berechnung der Herzleistung des Patienten ausgehend von dem Ausgangsmesswert.

32. Handgerät nach Anspruch 31, **gekennzeichnet durch** die kennzeichnenden Merkmale eines der vorhergehenden Ansprüche 12 bis 23 und 25 bis 30.
